# EUROPEAN PATENT APPLICATION

(11) **EP 2 428 511 A1**
(43) Date of publication of application: **14.03.2012**
(21) Application number: 11180758.2
(22) Date of filing: 09.09.2011
(51) Int. Cl.: C07D 307/80

(54) **Synthesis of dronedarone and salts thereof**

(30) Priority: 09.09.2010 IN MU25062010
(71) Applicant: USV Limited, Govandi, Mumbai 400 088 MAH (IN)
(72) Inventor: Sathe, Dhananjay Govind, 400 088 Mumbai (IN); Patil, Samadhan Daulat, 400 088 Mumbai (IN); Gaikwad, Umesh Dilip, 400 088 Mumbai (IN); Mantripragada, Narayana Rao, 400 088 Mumbai (IN); Shinde, Ajit Bhaskar, 400 088 Mumbai (IN)
(74) Representative: Schön, Christoph

(57) **Abstract**

The present invention relates to a process for preparation of Dronedarone or pharmaceutically acceptable salts thereof. More particularly, the present invention provides a process for preparation of Dronedarone hydrochloride, without the isolation of Dronedarone base.

## Description

### Field of invention:

The present invention relates to a process for preparation of Dronedarone or pharmaceutically acceptable salts thereof. More particularly, the present invention provides a process for preparation of Dronedarone hydrochloride, without the isolation of Dronedarone base.

### Background of the invention:

Dronedarone HCl is a benzofuran derivative with the following chemical name: N-{2-butyl-3- [4-(3-dibutylaminopropoxy)benzoyl]benzofuran-5-yl} methane sulfonamide, hydrochloride or 2-n-butyl-3-[4-(3-di-n-butylamino-propoxy) benzoyl]-5-methyl sulfonamido benzofuran hydrochloride represented by formula (I) below:

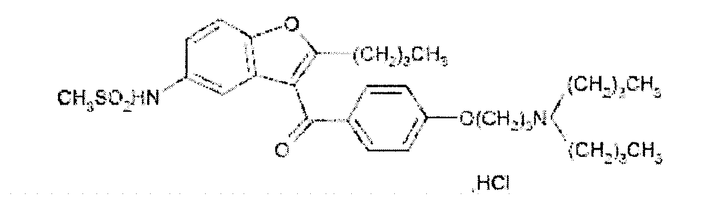

Dronedarone HCl is commercially marketed as MULTAQ® by Sanofi-Aventis. MULTAQ^{®} is available as film-coated tablet containing dronedarone hydrochloride equivalent to 400mg dronedarone for oral administration. The recommended dose of MULTAQ^{®} is one tablet taken twice a day, with morning and evening meals. MULTAQ^{®} is indicated to reduce the risk of cardiovascular hospitalization in patients with paroxysmal or persistent atrial fibrillation (AF) or atrial flutter (AFL), with a recent episode of AF/AFL and associated cardiovascular risk factors.

Dronedarone is an anti-arrhythmic agent, it works mainly by blocking channels through which charged particles of potassium move in and out of the muscle cells. The flow of charged particles in and out of the muscle cells produces the excessive electrical activity that leads to atrial fibrillation and rapid heart rate. By reducing the flow of potassium through channels, MULTAQ^{®} prevents the fibrillation from happening and lowers the heart rate.

US5223510 (hereinafter referred as US'510) discloses a process for preparation of Dronedarone comprising,
a) refluxing a mixture of 2-n-butyl-3-(4-hydroxy benzoyl)-5-nitro benzofuran (2) and 1-chloro-3-di-n-butylamino propane in methyl ethyl ketone in presence of potassium carbonate for 20 hours to get 2-n-butyl-3-[4-(3-di-n-butylamino-propoxy)benzoyl]-5-nitro benzofuran (3);
b) hydrogenating the obtained compound (3) in ethanol in presence of platinum oxide to get 5-amino-3-[4-(3-di-n-butylamino-propoxy)benzoyl]-2-n-butyl benzofuran (4);
c) treating the obtained compound (4) with methanesulfonyl chloride in dichloromethane in presence of triethylamine to get compound (1a) which is purified by column chromatography; and
d) dissolving the obtained compound (1a) in ethyl acetate and reacted with hydrogen chloride in ether to get Dronedarone hydrochloride (1).

This reaction is represented by Scheme 1 below, 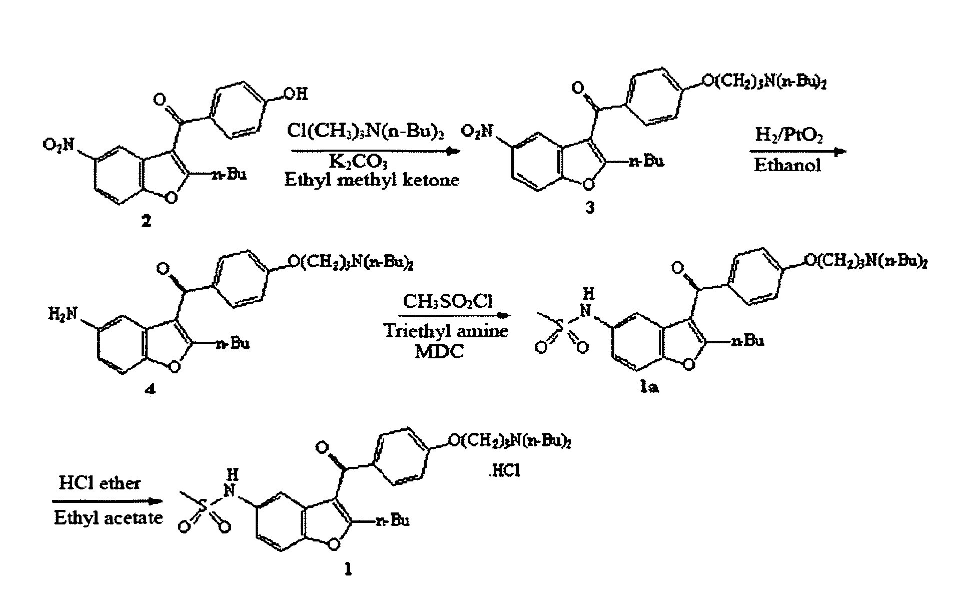 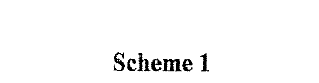 US'510 also discloses multistage process for preparation of the starting material, 2-n-butyl-3-(4-hydroxy benzoyl)-5-nitro benzofuran (2) as shown in Scheme 2, 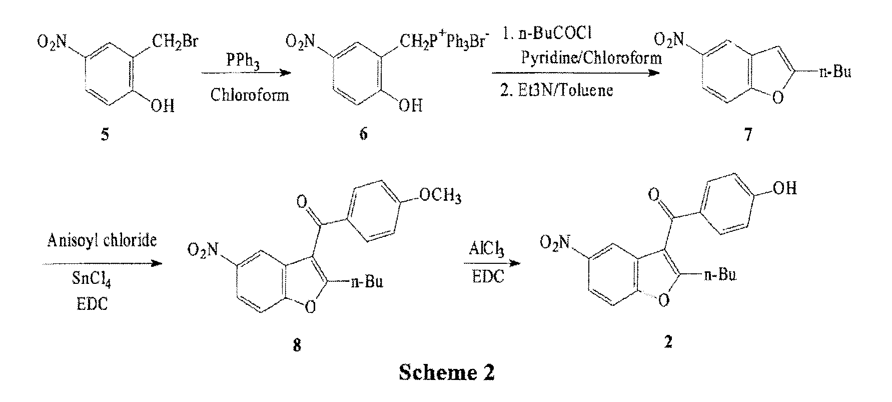 US6828448 discloses process for preparation of Dronedarone hydrochloride comprising the steps of;
a) hydrogenating 2-butyl-5-nitrobenzofuran in the presence of an appropriate catalyst to form 5-amino-2-butylbenzofuran;
b) reacting the obtained 5-amino-2-butylbenzofuran with methanesulfonyl chloride or methanesulfonic anhydride, in the presence of an acid acceptor, to form 2-butyl-5-(methanesulfonamido) benzofuran;
c) reacting the obtained 2-butyl-5-(methanesulfonamido) benzofuran in an organic phase consisting of one or more solvents chosen from halogenated or nonhalogenated hydrocarbons, with 4-[3-(dibutylamino)propoxy] benzoyl chloride hydrochloride, in presence of Lewis acid as catalyst, and then hydrolysis is carried out in order to obtain transiently, and without isolation, Dronedarone hydrochloride which is recovered in the organic phase. The hydrochloride formed is treated with a basic agent, giving Dronedarone. Dronedarone, thus obtained is treated with an organic or inorganic acid to form its pharmaceutically acceptable salt.
The disadvantages of the prior art processes are,
1. Synthesis of compound (3) is carried out in methyl ethyl ketone and requires 20 hours for completion.
2. The PtO₂ catalyst, used for reduction of compound (3), is very costly thus economically not viable.
3. The compound (1a) or Dronedarone base obtained by following US'510 produces the yields of 61%.
4. The purification of Dronedarone base is achieved by column chromatography which is not viable on a commercial scale.

In view of the above mentioned disadvantages, there exists a need to develop a simple and commercially viable process for the preparation of Dronedarone and its pharmaceutically acceptable salts. The present invention provides an industrially viable process for preparation of Dronedarone or pharmaceutically acceptable salt thereof, such as hydrochloride salt of Dronedarone in high yield and purity by overcoming the difficulties of the prior art. In particular, the present invention provides a process for preparation of Dronedarone hydrochloride without isolation of Dronedarone base.

### Object of the invention:

An object of the present invention is to provide simple, cost effective and industrially viable process for preparation of Dronedarone or pharmaceutically acceptable salts thereof.

Another object of the present invention is to provide Dronedarone or pharmaceutically acceptable salts thereof with purity more than 99.5%.

### Summary of the invention :

The present invention provides a process for preparation of Dronedarone or pharmaceutically acceptable salts thereof comprising the steps of,
a) condensing 2-n-butyl-3-(4-hydroxybenzoyl)-5-nitro benzofuran with 1-chloro-3-di-n-butylamino propane in presence of polar aprotic solvent other than methyl ethyl ketone to obtain 2-n-butyl 3-[4-(3-di-n-butylamino-propoxy)benzoyl]-5-nitro benzofuran;
b) converting the 2-n-butyl 3-[4-(3-di-n-butylamino-propoxy)benzoyl]-5-nitro benzofuran to Dronedarone or pharmaceutically acceptable salts thereof

Preferably, condensation in step a) is carried out at reflux temperature for 2 to 6 hours and said polar aprotic solvent is selected from acetonitrile, tetrahydrofuran, ethyl acetate, methyl acetate, dimethyl formamide, dimethyl acetamide or dimethyl sulfoxide.

Preferably, conversion in step b) comprises the steps of,
a) reducing the 2-n-butyl-3-[4-(3-di-n-butylamino-propoxy)benzoyl]-5-nitro benzofuran using a suitable reducing agent to obtain 5-amino-3-[4-(3-di-n-butylamino-propoxy)benzoyl]-2n-butyl- benzofuran;
b) treating the 5-amino-3-[4-(3-di-n-butylamino-propoxy)benzoyl]-2n-butyl benzofuran with methanesulfonyl chloride in absence of base optionally in presence of phase transfer catalyst to obtain Dronedarone hydrochloride.

Preferably, reduction in step a) is carried out using hydrogen in presence of catalyst other than platinum oxide in a suitable solvent selected from methanol, ethanol, n-propanol, isopropanol, n-butanol or mixture thereof, at temperature of 40 to 60°C and pressure of 3 to 6 kg/cm² for 2 to 5 hours. Preferably, the catalyst is Raney nickel.

Preferably, treatment in step b) is carried out in a solvent selected from toluene, xylene, n-pentane, cyclopentane, hexane, cyclohexane, 1,4-dioxane, chloroform or diethyl ether and phase transfer catalyst is R₄N⁺X⁻, where R represents -CH₃, -C₂H₅, -C₃H₇, -C₄H₉, C₆H₅CH₂- or combination thereof and where X⁻ represents Cl, Br, I or OH.

Another aspect of the present invention provides a process for preparation of

Dronedarone hydrochloride comprising the steps of,
a) treating 5-amino-3-[4-(3-di-n-butylamino-propoxy) benzoyl]-2n-butyl benzofuran with methanesulfonyl chloride in absence of base optionally in presence of phase transfer catalyst to obtain Dronedarone hydrochloride; and
b) optionally purifying the Dronedarone hydrochloride.

Preferably, Dronedarone hydrochloride is treated with salt forming agent selected from HCl, thionyl chloride or ammonium chloride to obtain Dronedarone hydrochloride with required hydrochloride content.

Another aspect of the present invention provides a process for preparation of Dronedarone hydrochloride wherein the process comprises treating Dronedarone hydrochloride with salt forming agent selected from HCl, thionyl chloride or ammonium chloride to obtain Dronedarone hydrochloride with required hydrochloride content.

Preferably, Dronedarone hydrochloride is purified by a process comprising the steps of,
a) treating dronedarone hydrochloride with a suitable solvent selected from methyl ethyl ketone, methyl isobutyl ketone, diethyl ketone, water or mixture thereof to obtain a solution; and
b) isolating pure Dronedarone hydrochloride from said solution.

Another aspect of the present invention provides a process for preparation of Dronedarone base comprising the steps of,
a) treating Dronedarone salt with suitable base selected from sodium hydroxide, potassium hydroxide, calcium hydroxide, cesium hydroxide, ammonia, sodium carbonate, potassium carbonate, sodium bicarbonate, potassium bicarbonate or mixture thereof in the presence of solvent selected from dichloromethane, chloroform, dichloroethane, water or mixture thereof, to obtain a mixture;
b) isolating Dronedarone base from said mixture;
c) optionally treating said Dronedarone base with solvent selected from hexane, pentane, cyclopentane, cyclohexane, benzene, toluene, 1,4-dioxane or diethyl ether.

Preferably, isolated Dronedarone base is characterized by X-ray diffraction pattern having peaks at 2-theta values of about 5.40, 8.11, 10.75, 10.95, 13.47, 16.54, 17.97, 19.07, 19.83, 20.44, 21.65, 22.48, 23.69, 24.37, 27.02, 27.75, 29.96 and 33.86 degrees.

### Brief description of the drawings:

Fig.1: X-ray diffraction pattern of Dronedarone obtained according to the present invention.
Fig.2: X-ray diffraction pattern of Dronedarone hydrochloride obtained according to the present invention.
Fig.3: DSC of Dronedarone hydrochloride obtained according to the present invention.

### Description of the invention:

The present invention provides an improved process for preparing Dronedarone or pharmaceutically acceptable salts thereof.

According to one embodiment of the present invention, there is provided a process for preparation of Dronedarone or pharmaceutically acceptable salts thereof which comprises the steps of,
a) condensing 2-n-butyl-3-(4-hydroxybenzoyl)-5-nitro benzofuran with 1-chloro-3-di-n-butylamino propane in presence of polar aprotic solvent other than methyl ethyl ketone to obtain 2-n-butyl 3-[4-(3-di-n-butylamino-propoxy)benzoyl]-5-nitro benzofuran; and
b) converting 2-n-butyl-3-[4-(3-di-n-butylamino-propoxy)benzoyl]-5-nitro benzofuran to Dronedarone or pharmaceutically acceptable salts thereof. Polar aprotic solvent is selected from acetonitrile, acetone, tetrahydrofuran (THF), ethyl acetate, methyl acetate, dimethyl formamide, dimethylacetamide, dimethyl sulfoxide (DMSO), preferably acetonitrile.

According to a preferred embodiment of the present invention, there is provided a process for preparation of Dronedarone or pharmaceutically acceptable salts thereof comprising condensation of 2-n-butyl-3-(4-hydroxy benzoyl)-5-nitro benzofuran with 1-chloro-3-di-n-butylamino propane in presence of acetonitrile and base selected from potassium carbonate, sodium carbonate, sodium hydroxide, potassium hydroxide, calcium hydroxide, cesium hydroxide or mixture thereof, preferably potassium carbonate at reflux temperature for 2-6 hours, preferably for 3-4 hours. After the completion of reaction, the reaction mixture is cooled to room temperature. The reaction mixture is filtered and the residue is washed with acetonitrile. The filtrate is concentrated to obtain an oily mass. The obtained oily mass is stirred with about 3-7%, preferably 5% NaOH solution for 5-15 min, preferably for 10 min. The 2-n-butyl-3-[4-(3-di-n-butylamino-propoxy)benzoyl]-5-nitro benzofuran is extracted with an ether selected from methyl tert butyl ether(MTBE), diethyl ether, 1,4-dioxane, tetrahydrofuran(THF), dimethoxyethane(DME), diethoxyethane or mixture thereof, preferably methyl tert butyl ether. The ether extract is dried over anhydrous sodium sulphate and concentrated to obtain 2-n-butyl 3-[4-(3-di-n-butylamino-propoxy)benzoyl]-5-nitro benzofuran as an oil, which can be used for the next step without further purification. The obtained oily product is then converted to Dronedarone or pharmaceutically acceptable salts thereof, preferably hydrochloride.

Prior art discloses the use of methyl ethyl ketone in the synthesis of 2-n-butyl-3-[4-(3-di-n-butylamino-propoxy)benzoyl]-5-nitro benzofuran. Use of methyl ethyl ketone takes nearly 20 hours for the completion of reaction. It was surprisingly found by inventors of the present invention that when acetonitrile is used as the solvent for condensation, the reaction time is reduced from 20 hrs to 3 - 4 hours. The solvent acetonitrile is chosen over the ketone on the basis of polarity. Acetonitrile is more polar than ketone. The use of acetonitrile increases the rate of reaction and the reaction tends to complete within 3 to 4 hrs and provides 2-n-butyl 3-[4-(3-di-n-butylamino-propoxy)benzoyl]-5-nitro benzofuran in high yield and purity. The obtained compound (3) can be used for further reaction without any purification.

Another embodiment of the present invention provides conversion of 2-n-butyl-3-[4-(3-di-n-butylamino-propoxy)benzoyl]-5-nitro benzofuran to Dronedarone hydrochloride comprising the steps of,
a) reducing 2-n-butyl-3-[4-(3-di-n-butylamino-propoxy) benzoyl]-5-nitro benzofuran using a suitable reducing agent to obtain 5-amino-3-[4-(3-di-n-butylamino-propoxy)benzoyl]-2-n-butyl benzofuran;
b) treating 5-amino-3-[4-(3-di-n-butylamino-propoxy)benzoyl]-2-n-butyl benzo furan with methanesulfonyl chloride in absence of base optionally in presence of phase transfer catalyst to obtain Dronedarone hydrochloride; and
c) optionally treating Dronedarone hydrochloride with salt forming agent to obtain Dronedarone hydrochloride with the required HCl content.

Suitable reducing agent is selected from hydrogen in presence of catalyst other than platinum oxide, lithium aluminium hydride, Sn/HCl or Fe/HCl. Catalyst is selected from Raney nickel, palladium or rhodium on supports such as carbon, silica or alumina; or oxides thereof.

R₄N⁺X⁻ 10

R = CH₃, C₂H₅, C₃H₇, C₄H₉, C₆H₅CH₂-

or combination thereof

X = Cl, Br, I, OH

Suitable phase transfer catalyst used is selected from formula (10),

In a preferred embodiment, reduction of 2-n-butyl-3-[4-(3-di-n-butylamino-propoxy)benzoyl]-5-nitro benzofuran is carried out using hydrogen in presence of Raney nickel in solvent selected from methanol, ethanol, n-propanol, isopropanol, n-butanol, preferably methanol. 2-n-butyl-3-[4-(3-di-n-butylamino-propoxy)benzoyl]-5-nitro benzofuran is subjected to hydrogenation in presence of Raney nickel catalyst under pressure of 3 to 6 Kg/cm2, preferably 4 to 5 Kg/cm2 at temperature of about 40- 60°C, preferably 45 to 50°C for 2-5 hours, preferably 3-4 hours. After the completion of reaction, the reaction mixture is filtered through hyflo bed and washed with methanol. The obtained filtrate is concentrated to get 5-amino-3-[4-(3-di-n-butylamino-propoxy)benzoyl]-2-n-butyl benzofuran as an oil, which can be used for the next step without further purification.

The obtained 5-amino-3-[4-(3-di-n-butylamino-propoxy)benzoyl]-2-n-butyl benzofuran is treated with methanesulfonyl chloride in absence of base and in presence of tetramethyl ammonium chloride to get Dronedarone hydrochloride. The reaction is carried out by refluxing a mixture of 5-amino-3-[4-(3-di-n-butylamino-propoxy)benzoyl]-2-n-butyl benzofuran and tetramethyl ammonium chloride in a suitable solvent selected from toluene, xylene, n-pentane, cyclopentane, hexane, cyclohexane, 1,4-dioxane, chloroform or diethyl ether, preferably toluene for 30 - 90 minutes, preferably for 60 min to obtain a reaction mixture. A solution of methane sulfonyl chloride in solvent selected from toluene, xylene, n-pentane, cyclopentane, hexane, cyclohexane, 1,4-dioxane, chloroform or diethyl ether, preferably toluene, is added slowly to the refluxed reaction mixture in about 10-20min, preferably 15 minutes. After the completion of reaction, the reaction mixture is cooled to 50 to 65°C, preferably 55 to 60°C to obtain an oily residue. The obtained oily residue is separated by decantation. The obtained oily residue is quenched in water. The reaction mixture is stirred at room temperature for 24 hours. The obtained solid is filtered, washed and dried to obtain Dronedarone hydrochloride.

Prior art teaches the use of platinum oxide as catalyst for hydrogenation. The advantage of using Raney Nickel as reducing catalyst is that it is cheaper in comparison with other catalyst and provides product in good yield and purity. The use of Raney Nickel makes the process cost effective and commercially viable. The oily product obtained, if required can be used as such for the next step or can be further purified by formation of suitable salt such as oxalate, hydrochloride or the like.

Prior art discloses the reaction of 5-amino-3-[4-(3-di-n-butylamino-propoxy)benzoyl]-2-n-butyl benzofuran with methanesulfonyl chloride in the presence of base to produce dronedarone base. Dronedarone base is then converted to its pharmaceutically acceptable salts. The present invention carries the reaction of 5-amino-3-[4-(3-di-n-butylamino-propoxy)benzoyl]-2-n-butyl benzofuran with methanesulfonyl chloride in the absence of base to produce Dronedarone hydrochloride directly. Thus the process of the present invention does not involves isolation of Dronedarone base thereby reducing the number of the steps for obtaining the desired product as compared to the prior art. Thus, it makes the process simple, cost effective and suitable for industrial scale up. The obtained Dronedarone hydrochloride is then treated with salt forming agent selected from HCl, thionyl chloride or ammonium chloride to obtain Dronedarone hydrochloride with the required HCl content.

Another embodiment of the present invention provides a process for preparation of Dronedarone hydrochloride comprising the steps of,
a) treating 5-amino-3-[4-(3-di-n-butylamino-propoxy) benzoyl]-2n-butyl benzofuran with methanesulfonyl chloride in absence of base optionally in presence of phase transfer catalyst to obtain Dronedarone hydrochloride;
b) optionally purifying Dronedarone hydrochloride.

In a preferred embodiment, 5-amino-3-[4-(3-di-n-butylamino-propoxy)benzoyl]-2-n-butyl benzofuran is treated with methanesulfonyl chloride in absence of base and in presence of tetramethyl ammonium chloride to get Dronedarone hydrochloride. The reaction is carried out by refluxing a mixture of 5-amino-3-[4-(3-di-n-butylamino-propoxy)benzoyl]-2-n-butyl benzofuran and tetramethyl ammonium chloride in a suitable solvent selected from toluene, xylene, n-pentane, cyclopentane, hexane, cyclohexane, 1,4-dioxane, chloroform or diethyl ether, preferably toluene for 30 - 90 minutes, preferably for 60 min to obtain a reaction mixture. A solution of methane sulfonyl chloride in solvent selected from toluene, xylene, n-pentane, cyclopentane, hexane, cyclohexane, 1,4-dioxane, chloroform or diethyl ether, preferably toluene, is added slowly to the refluxed reaction mixture in about 10-20min, preferably in 15 minutes. After the completion of reaction, the reaction mixture is cooled to 50 to 65°C, preferably 55 to 60°C to obtain an oily residue. The obtained oily residue is separated by decantation. The obtained oily residue is quenched in water. The reaction mixture is stirred at room temperature for 24 hours. The obtained solid is filtered, washed and dried to obtain Dronedarone hydrochloride.

An alternate embodiment of the present invention provides a process for preparation of Dronedarone hydrochloride comprises reacting 5-amino-3-[4-(3-di-n-butylamino-propoxy)benzoyl]-2-n-butyl benzofuran with methanesulfonyl chloride in the absence of base and in the absence of phase transfer catalyst to obtain Dronedarone hydrochloride.

In a preferred embodiment, a mixture of 5-amino-3-[4-(3-di-n-butylamino-propoxy)benzoyl]-2-n-butyl benzofuran and a suitable solvent selected toluene, benzene, pentane, cyclopentane, hexane, cyclohexane, 1,4-dioxane, chloroform or diethyl ether, preferably toluene are taken in round bottom flask and refluxed for 15 min at the same temperature. A solution of methane sulfonyl chloride in solvent selected from toluene, xylene, n-pentane, cyclopentane, hexane, cyclohexane, 1,4-dioxane, chloroform or diethyl ether, preferably toluene is added to the above mixture. The reaction was monitored by TLC. After completion of reaction, the reaction mixture is cooled to room temperature followed by addition of water and ester selected from ethyl acetate, methyl acetate or butyl acetate, preferably ethyl acetate. The reaction mixture is stirred for 30 min and the layers are separated. The aqueous layer is extracted with ester solvent. The combined organic layers are dried over anhydrous sodium sulfate and concentrated to obtain Dronedarone hydrochloride.

Another embodiment of the present invention provides Dronedarone hydrochloride with required HCl content comprising,
a) treating Dronedarone hydrochloride with salt forming agent selected from HCl, thionyl chloride or ammonium chloride to obtain Dronedarone hydrochloride with required HCl content; and
b) optionally purifying Dronedarone hydrochloride.

In a preferred embodiment, Dronedarone hydrochloride is treated with salt forming agent selected from HCl, thionyl chloride or ammonium chloride, preferably HCl at temperature of about 40-60°C, preferably 45 to 50°C for 1 to 4 hours, preferably for 2 hours to get a mixture. The obtained hot mixture is filtered and the wet cake is washed with hot water. The obtained wet cake is dried at 50-70°C, preferably at 60-65°C to obtain Dronedarone hydrochloride with required HCl content.

Another embodiment of the present invention provides a process for purification of Dronedarone hydrochloride comprising,
a) treating Dronedarone hydrochloride with a suitable solvent to obtain a solution; and
b) isolating pure Dronedarone hydrochloride from said solution.

Suitable solvent used for purification is selected from ketone, water or mixture thereof. Ketone is selected from methyl ethyl ketone, acetone, methyl isobutyl ketone, diethyl ketone, preferably methyl ethyl ketone.

In a preferred embodiment, the present invention provides the purification of Dronedarone hydrochloride by treating Dronedarone hydrochloride with methyl ethyl ketone at reflux temperature to obtain a solution. The obtained solution is filtered through hyflo bed and the filtrate is cooled to 20 to 30°C. This solution is further cooled to 10-25°C, preferably 15-20°C and maintained at the same temperature for 1-3 hours, preferably for 2 hours to obtain a solid. The obtained solid is filtered and washed with methyl ethyl ketone to obtain pure Dronedarone hydrochloride. The obtained Dronedarone hydrochloride is optionally subjected to further purification. The process of purification is preferably repeated in order to improve the colour and texture of the final product.

Prior art discloses the use of acetone for the purification of dronedarone hydrochloride. It has been found that using methyl ethyl ketone improves the yield of the desired product. The yield obtained by using acetone for purification of dronedarone hydrochloride is about 70% and the yield obtained by using methyl ethyl ketone is about 80%.

Pure Dronedarone hydrochloride obtained according to present invention is substantially pure and has chemical purity of more than 99.5% with all known impurities below 0.15% and unknown impurities below 0.1%.

Another embodiment of the present invention provides Dronedarone hydrochloride having all genotoxic impurities, well controlled below the allowable limit of 16 ppm for daily dose of 800 mg.

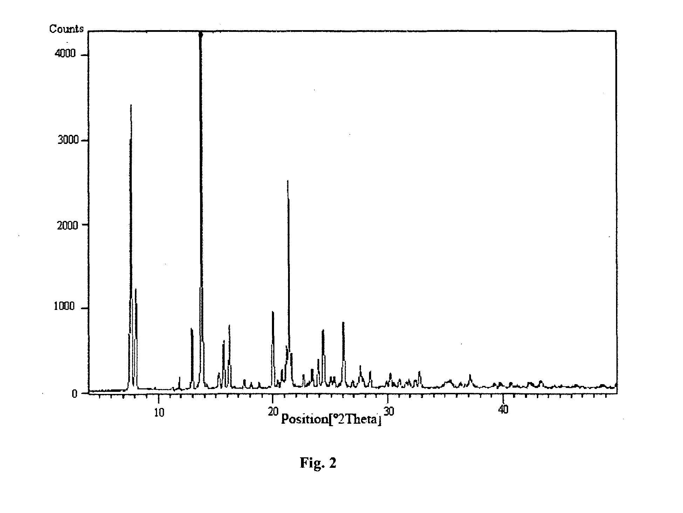

Dronedarone hydrochloride obtained according to the present invention is characterized by X-ray diffraction pattern as shown in Fig. 2. It is further characterized by X-ray diffraction pattern having peaks expressed as 2-theta values of about 7.67, 11.85, 13.00, 13.82, 15.26, 15.72, 16.21, 17.48, 18.07, 18.77, 20.01, 20.39, 20.76, 21.19, 21.38, 21.61, 22.63, 23.38, 23.88, 24.33, 24.97, 25.26, 26.09, 26.91, 27.54, 28.41, 29.86, 30.24, 31.04, 31.87, 32.41, 32.76, 35.50, 36.30, 37.14, 42.28 and 43.28 degrees.

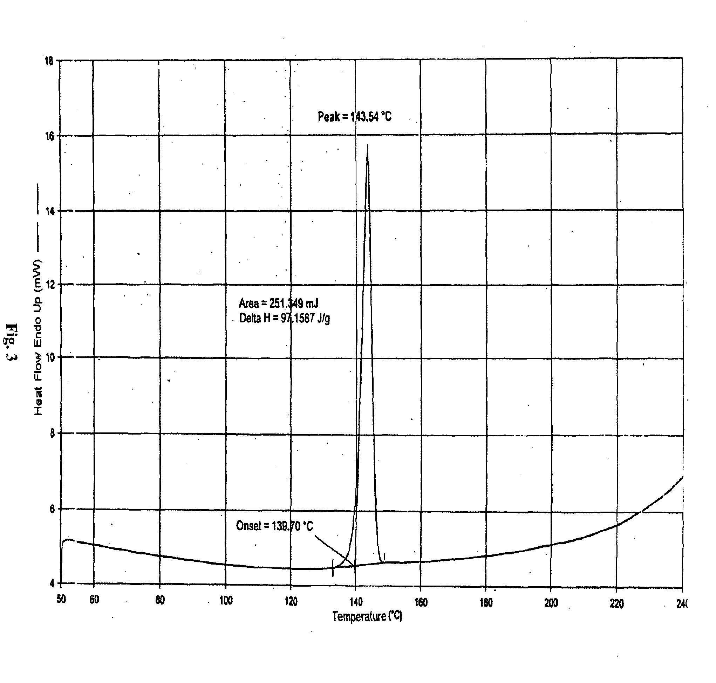

Dronedarone hydrochloride obtained according to present invention is characterized by Differential Scanning Calorimetry (DSC) thermagram having one endothermic peak at 143.54°C, as shown in Fig. 3.

Another embodiment of the present invention provides the process for preparation of dronedarone base comprising the steps of,
a) treating Dronedarone salt with suitable base in presence of solvent to obtain a mixture;
b) isolating Dronedarone base from said mixture;
c) optionally treating Dronedarone base with solvent selected from hexane, pentane, cyclopentane, cyclohexane, benzene, toluene, 1,4-dioxane, chloroform or diethyl ether.

In preferred embodiment, the present invention provides the process of isolation of dronedarone base by treating dronedarone hydrochloride with a suitable base selected from sodium hydroxide, potassium hydroxide, calcium hydroxide, cesium hydroxide, ammonia, sodium carbonate, potassium carbonate, sodium bicarbonate, potassium bicarbonate or the like in presence of a solvent to obtain a mixture. The obtained mixture is stirred at 0-10°C, preferably 0-5°C for 30 - 90 minutes, preferably 60 min. A halogenated solvent selected from dichloromethane, dichloroethane, chloroform or the like is added to the mixture. The layers are separated and the organic layer is concentrated to obtain a residue. The obtained residue is treated with suitable solvent such as hexane, pentane, cyclopentane, cyclohexane, benzene, toluene, 1,4-dioxane or diethyl ether to obtain Dronedarone base.

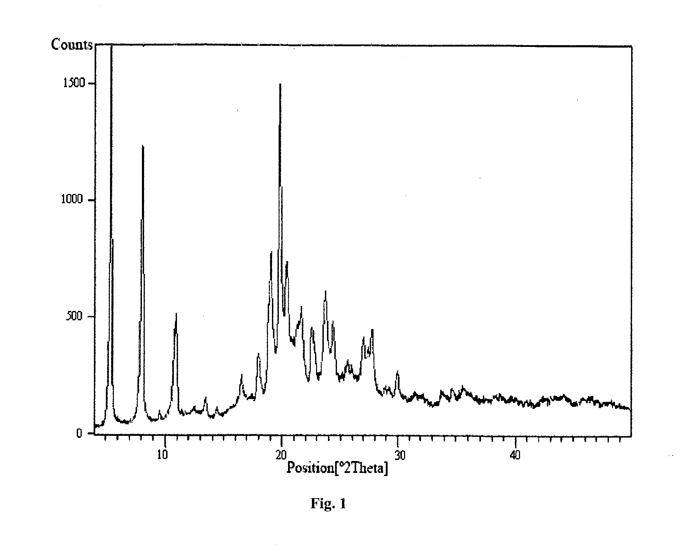

Another embodiment of present invention provides Dronedarone base obtained according to the present invention characterized by X-ray diffraction pattern as shown in Fig.1. It is further characterized by X-ray diffraction pattern having peaks expressed as 2-theta values at about 5.40, 8.11, 10.75, 10.95, 13.47, 16.54, 17.97, 19.07, 19.83, 20.44, 21.65, 22.48, 23.69, 24.37, 27.02, 27.75, 29.96 and 33.86 degrees.

The process for preparation of Dronedarone hydrochloride according to the present invention is represented in scheme below,

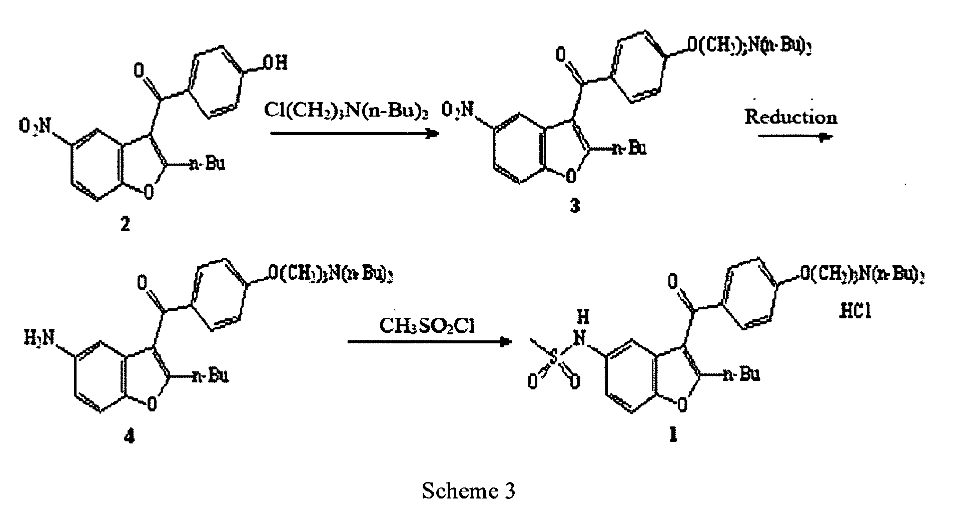

This present invention consistently provides Dronedarone or salt thereof particularly, Dronedarone hydrochloride in high yield and ICH purity as compared to the prior art. Thus the process as discussed in the present invention yields Dronedarone hydrochloride having all known and unknown impurities below 0.15 %, preferably below 0.1 %.

Dronedarone hydrochloride obtained according to the present invention exhibits the following particle size distribution, d(0.9) less than or equal to about 200 µm, d(0.5) less than or equal to about 50 µm and d(0.1) less than or equal to about 20 µm. Preferably, d(0.9) is less than or equal to about 100 µm, more preferably less than or equal to about 50 µm, most preferably less than or equal to about 20 µm.

Dronedarone hydrochloride obtained according to present invention may be micronized using conventional micronization techniques.

Another embodiment of the present invention provides pharmaceutical composition comprising Dronedarone hydrochloride or any polymorph thereof prepared according to the present invention and at least one pharmaceutically acceptable excipient, used in therapy such as in a method of treating atrial fibrillation or atrial flutter. The pharmaceutical compositions may be prepared by any conventional techniques known in the art.

2-n-butyl-3-(4-hydroxybenzoyl)-5-nitro benzofuran used in the synthesis of Dronedarone hydrochloride can be synthesized by any process known in the art or by a process comprising the steps of,
a) condensing 2-hydroxy 5-nitro benzaldehyde with methyl 2-bromo pentanoate in presence of base and suitable solvent to obtain 2-n-butyl 5-nitro benzofuran; and
b) reacting obtained 2-butyl-5-nitrobenzofuran with 4-methoxy benzoyl chloride under Friedel-Crafts reaction conditions followed by demethylation to form 2-butyl-3-(4-methoxybenzoyl)-5-nitrobenzofuran.

1-chloro-3-di-n-butylamino propane, used in the synthesis of Dronedarone hydrochloride can be prepared by any process known in the art or by a process comprising the steps of,
a) treating di-n-butylamine with 3-chloropropanol to obtain 1-hydroxy-3-di-n-dibutylamino propane;
b) chlorinating 1-hydroxy-3-di-n-dibutylamino propane to obtain 1-chloro-3-di-n-butylamino propane.

Unless otherwise indicated, the following definitions are set forth to illustrate and define the meaning and scope of the various terms used to describe the invention herein.

The term "substantially pure" means a compound having less than about 1%, preferably less than about 0.5% more preferably less than about 0.3%, most preferably less than about 0.15% of undesired impurities or other polymorphic forms.

The term "reflux temperature" means the temperature at which the solvent or solvent system reflux or boils at atmospheric pressure.

The term "room temperature" means a temperature from about 10°C to 45°C, preferably 25°C to 30°C.

Identification of solid obtained by the present invention can be made by methods known in the art, such as X-Ray powder diffraction (XRPD), Fourier Transform Infrared (FT-IR) spectra, and differential scanning calorimetry (DSC).

The DSC and XRPD methods used for the identification and characterization of Dronedarone or salt thereof are described below:
a) X-ray powder diffraction : XRPD Spectrum was recorded on PANalytical X'pert Pro diffractometer equipped with accelerator detector using Copper Kα (λ = 1.5406 Å) radiation with scanning range between 4-50 2θ at scanning speed af 2°/min.
b) DSC : DSC analysis was recorded on Perkin Elmer at a heating rate of 20°C per minute at a temperature range from 50°C to 250°C.

The following examples are for illustrative purposes only and are not intended, nor should they be interpreted, to limit the scope of the invention in any manner.

### Examples:

### Example 1:

### Preparation of 2-n-butyl-3-[4-(3-di-n-butylamino-propoxy)benzoyl]-5-nitro benzofuran:

A mixture of 2-n-butyl-3-(4-hydroxybenzoyl)-5-nitro benzofuran (119 gm, 0.35 mol) and 48 gm of potassium carbonate in 600 ml of acetonitrile was stirred for 30 min and 1-chloro-3-di-n-butylamino propane (72 gm, 0.35 mol) was added to the mixture. The reaction mixture was heated to reflux for 3 to 4 hr. After completion of the reaction, the mixture was cooled to room temperature. The mixture was filtered and the residue was washed with acetonitrile. The collected filtrate was concentrated to obtain an oily mass. The obtained mass was treated with 5% NaOH (2.5 L) for 10 min followed by extraction with methyl tert butyl ether (MTBE) (2.5 L). The organic layer was dried over sodium sulfate and concentrated to get 162 gm of oily product.

Yield: 91%.

### Example 2:

### Preparation of 5-amino-3-[4-(3-di-n-butylamino-propoxy)benzoyl]-2-n-butyl benzofuran:

2-n-butyl-3-[4-(3-di-n-butylamino-propoxy)benzoyl]-5-nitro benzofuran (155 gm, 0.31 mol) in methanol (6.2 lit) was subjected to hydrogenation in presence of Raney nickel (31 gm) in a hydrogenation apparatus under pressure of about 5kg/cm² of hydrogen at room temperature for 3-4 hours. The completion of reaction was monitored by TLC. The reaction mixture was then filtered through hyflo bed and washed with methanol. The filtrate was concentrated to obtain 143 gm of titled product as an oil.
Yield: 98%.

### Example 3:

### Preparation of 5-amino-3-[4-(3-di-n-butylamino-propoxy)benzoyl]-2-n-butyl benzofuran:

2-n-butyl-3-[4-(3-di-n-butylamino-propoxy)benzoyl]-5-nitro benzofuran (155 gm, 0.31 mol) in methanol (3.1 lit) was subjected to hydrogenation in presence of Raney nickel (31 gm) in a hydrogenation apparatus under pressure of about 5kg/cm² of hydrogen and temperature of 45-50°C for 3-4 hours. The completion of reaction was monitored by TLC. The reaction mixture was filtered through hyflo bed and the residue was washed with methanol. The filtrate was concentrated to get 143 gm of titled product.
Yield: 98%

### Example 4:

### Preparation of 2-n-butyl 3-[4-(3-di-n-butylamino-propoxy)benzoyl] 5-methylsulfonamido benzofuran hydrochloride (Dronedarone hydrochloride):

A mixture of 5-amino-3-[4-(3-di-n-butylamino-propoxy)benzoyl]-2-n-butyl benzofuran (150 gm, 0.313 mol) and tetramethyl ammonium chloride (8.6 gm, 0.078 mol) in toluene (1.2 lit) was refluxed for I hr. A solution of methanesulfonyl chloride (71.7 gm, 0.626 mol) in toluene (300 ml) was added slowly to the refluxed reaction mixture in 15 min. The reaction was monitored by TLC. After completion of the reaction, the mixture was cooled to 55 to 60°C to obtain brown oily residue. The obtained brown oily residue was separated by decantation of toluene. The obtained oily residue was added to water (1.5 lit) under stirring. The reaction mixture was stirred at room temperature for 24 hours and filtered to obtain a solid. The obtained solid was washed with water (3 x 600 ml) and dried to obtain 174 gm of Dronedarone hydrochloride.
Yield: 94%.

### Example 5:

### Preparation of 2-n-butyl-3-[4-(3-di-n-butylamino-propoxy)benzoyl]-5-methyl sulfonamido benzofuran hydrochloride (Dronedarone hydrochloride):

5-amino-3-[4-(3-di-n-butylamino-propoxy)benzoyl]-2-n-butyl benzofuran (150 gm, 0.313 mol) and toluene (1.2 lit) were taken in round bottom flask and refluxed for 15 minutes. A solution of methanesulfonyl chloride (71.7 gm, 0.626 mol) in toluene (300 ml) was added to the above mixture at reflux temperature. The reaction was monitored by TLC. After completion of reaction, the mixture was cooled to room temperature followed by addition of water (4.5 lit) and ethyl acetate(2.6 lit). The mixture was stirred for 30 min and the organic layer was separated. The aqueous layer was extracted with ethyl acetate (2 x 1.3 lit). The combined organic layers were dried over anhydrous sodium sulfate and concentrated to get 165 gm of crude Dronedarone hydrochloride.
Yield: 89%.

### Example 6:

### Purification of Dronedarone hydrochloride:

The crude Dronedarone hydrochloride (150 gm) was suspended in water (2.40 lit) and 32 ml of conc. HCl was added to the suspension. The reaction mixture was heated at 45-50°C for 2 hours. The hot mixture was filtered and the wet cake was washed with hot water. The obtained wet cake was dried at 60-65°C to yield 142 gm of Dronedarone hydrochloride.
Yield: 95%; Purity: 98.7%

### Example 7:

### Purification of Dronedarone hydrochloride from acetone:

Dronedarone hydrochloride (150 gm) was dissolved in acetone (2.1 lit) at reflux temperature and 7 gm of charcoal was added to the obtained solution. The refluxed solution was filtered and the filtrate was allowed to cool to 20-25°C to obtain a solid. The obtained solid was filtered, washed with acetone and dried to obtain 105 gm of pure off-white crystalline solid of dronedarone hydrochloride.
Yield: 70% ; Purity: 99.2%

### Example 8:

### Purification of Dronedarone hydrochloride from Methyl ethyl ketone:

Dronedarone hydrochloride (150 gm) was dissolved in methyl ethyl ketone (1.05 lit) at reflux temperature and 7 gm of charcoal was added to the obtained solution. The refluxed solution was filtered and the filtrate was allowed to cool to 15-20°C to obtain a solid. The obtained solid was filtered, washed with methyl ethyl ketone and dried at 60-65°C to obtain 120 gm of pure white crystalline solid dronedarone hydrochloride.
Yield: 80%; Purity: 99.5%

### Example 9:

### Preparation of Dronedarone base:

5 gm of Dronedarone hydrochloride was added to cold solution of sodium hydroxide (40ml,1M) to obtain a mixture. The mixture was stirred at 0-5°C for 1hr. 25ml of dichloromethane (MDC) was added to the mixture. The layers were separated and the organic layer was concentrated to obtain a residue. The obtained residue was treated with hexane to get 4.2 gm of Dronedarone base.

## Claims

1. A process for preparation of Dronedarone or pharmaceutically acceptable salts thereof comprising,
a) condensing 2-n-butyl-3-(4-hydroxybenzoyl)-5-nitro benzofuran with 1-chloro-3-di-n-butylamino propane in presence of polar aprotic solvent other than methyl ethyl ketone to obtain 2-n-butyl 3-[4-(3-di-n-butylamino-propoxy)benzoyl]-5-nitro benzofuran;
b) converting said 2-n-butyl 3-[4-(3-di-n-butylamino-propoxy)benzoyl]-5-nitro benzofuran to Dronedarone or pharmaceutically acceptable salts thereof.

2. The process as claimed in claim 1, wherein said condensation in step a) is carried out at reflux temperature for 2 to 6 hours and said polar aprotic solvent is selected from acetonitrile, acetone, tetrahydrofuran, ethyl acetate, methyl acetate, dimethyl formamide, dimethyl acetamide or dimethyl sulfoxide.

3. The process as claimed in claim 1, wherein said conversion in step b) comprises the steps of,
a) reducing said 2-n-butyl-3-[4-(3-di-n-butylamino-propoxy)benzoyl]-5-nitro benzofuran using a suitable reducing agent to obtain 5-amino-3-[4-(3-di-n-butylamino-propoxy)benzoyl]-2n-butyl- benzofuran;
b) treating said 5-amino-3-[4-(3-di-n-butylamino-propoxy)benzoyl]-2n-butyl benzofuran with methanesulfonyl chloride in absence of base optionally in presence of phase transfer catalyst to obtain Dronedarone hydrochloride.

4. The process as claimed in claim 3, wherein said reduction in step a) is carried out using hydrogen in presence of catalyst other than platinum oxide in a suitable solvent selected from methanol, ethanol, n-propanol, isopropanol, n-butanol or mixture thereof, at temperature of 40 to 60°C and pressure of 3 to 6 kg/cm² for 2 to 5 hours.

5. The process as claimed in claim 4, wherein said catalyst is Raney nickel

6. The process as claimed in claim 3, wherein said treatment in step b) is carried out in a solvent selected from toluene, xylene, n-pentane, cyclopentane, hexane, cyclohexane, 1,4-dioxane, chloroform or diethyl ether; and said phase transfer catalyst is R₄N⁺X⁻, where R represents -CH₃, - C₂H₅, -C₃H₇, -C₄H₉, C₆H₅CH₂- or combination thereof and where X⁻ represents Cl, Br, I or OH.

7. A process for preparation of Dronedarone hydrochloride comprising the steps of,
a) treating 5-amino-3-[4-(3-di-n-butylamino-propoxy) benzoyl]-2n-butyl benzofuran with methanesulfonyl chloride in absence of base optionally in presence of phase transfer catalyst to obtain Dronedarone hydrochloride; and
b) optionally purifying said Dronedarone hydrochloride.

8. The process as claimed in claim 3 or claim 7, wherein said Dronedarone hydrochloride is treated with salt forming agent selected from HCl, thionyl chloride or ammonium chloride to obtain Dronedarone hydrochloride with required hydrochloride content.

9. A process for preparation of Dronedarone hydrochloride wherein the process comprises treating Dronedarone hydrochloride with salt forming agent selected from HCl, thionyl chloride or ammonium chloride to obtain Dronedarone hydrochloride with required hydrochloride content.

10. The process as claimed in claim 7 or claim 8, wherein said Dronedarone hydrochloride is purified by a process comprising the steps of,
a) treating dronedarone hydrochloride with a suitable solvent selected from methyl ethyl ketone, methyl isobutyl ketone, diethyl ketone, water or mixture thereof to obtain a solution; and
b) isolating pure Dronedarone hydrochloride from said solution.

11. A process for preparation of Dronedarone base comprising the steps of,
a) treating Dronedarone salt with suitable base selected from sodium hydroxide, potassium hydroxide, calcium hydroxide, cesium hydroxide, ammonia, sodium carbonate, potassium carbonate, sodium bicarbonate, potassium bicarbonate or mixture thereof in the presence of solvent selected from dichloromethane, chloroform, dichloroethane, water or mixture thereof, to obtain a mixture;
b) isolating Dronedarone base from said mixture;
c) optionally treating said Dronedarone base with solvent selected from hexane, pentane, cyclopentane, cyclohexane, benzene, toluene, 1,4-dioxane, chloroform or diethyl ether.

12. The process as claimed in claim 11, wherein said isolated Dronedarone base is **characterized by** X-ray diffraction pattern having peaks at 2-theta values of about 5.40, 8.11, 10.75, 10.95, 13.47, 16.54, 17.97, 19.07, 19.83, 20.44, 21.65, 22.48, 23.69, 24.37, 27.02, 27.75, 29.96 and 33.86 degrees.
